# EUROPÄISCHE PATENTSCHRIFT

(11) **EP 3 510 084 B1**
(45) Veröffentlichungstag und Bekanntmachung des Hinweises auf die Patenterteilung: **03.03.2021**
(21) Anmeldenummer: 17758118.8
(22) Anmeldetag: 21.08.2017
(51) Int. Cl.: C08J 3/24, C08J 7/12, A61L 27/16, A61L 27/50, A61K 31/355, C08K 5/1545

(54) **VERFAHREN ZUR HERSTELLUNG EINES VERNETZTEN FORMKÖRPERS AUS UHMWPE**
METHOD FOR PRODUCING A CROSS-LINKED MOULDED BODY FROM UHMWPE
PROCÉDÉ DE FABRICATION D'UN CORPS MOULÉ RÉTICULÉ À PARTIR DE UHMWPE

(30) Priorität: 06.09.2016 DE 102016116597
(43) Veröffentlichungstag der Anmeldung: 17.07.2019
(73) Patentinhaber: Aesculap AG, 78532 Tuttlingen (DE)
(72) Erfinder: MULLIEZ, Marie Anne, 78532 Tuttlingen (DE); REU, Gerhard, 78532 Tuttlingen (DE)
(74) Vertreter: Hoeger, Stellrecht & Partner Patentanwälte mbB
(86) Internationale Anmeldenummer: PCT/EP2017/070989
(87) Internationale Veröffentlichungsnummer: WO 2018/046283

(56) Entgegenhaltungen:
- WO-A1-2007/019874
- Hans Hartmann: "Sterilisations-Methoden - Alternativen zumGammastrahlen", , 30. April 2015 (2015-04-30), Seiten 1-39, XP002774758, Gefunden im Internet: URL:http://www.saq.ch/fileadmin/user_uploa d/veranstaltungen/referate/741/1_gamma_har tmann.pdf [gefunden am 2017-10-17]

## Beschreibung

Die vorliegende Erfindung betrifft ein Verfahren zur Herstellung eines vernetzten Formkörpers aus UHMWPE, umfassend die Schritte:
- Bereitstellen eines Formkörpers aus UHMWPE, welches mit einem Antioxidans versetzt ist;
- Aufheizen des Formkörpers auf eine Temperatur von 100 °C oder mehr; und
- Bestrahlen des Formkörpers, um das UHMWPE in dem Formkörper zu vernetzen.

Die Erfindung betrifft ferner ein Verfahren zur Herstellung eines Implantats oder eines Implantatteils, umfassend die Durchführung des vorstehend beschriebenen Verfahrens und die zerspanende Bearbeitung des vernetzten Formkörpers.

Ultrahochmolekulares Polyethylen (UHMWPE) ist ein Kunststoffmaterial, das aufgrund seiner hervorragenden mechanischen Eigenschaften seit Längerem für die Herstellung von orthopädischen Implantaten, insbesondere von Inlays für künstliche Hüftgelenke oder andere Gelenkprothesen, eingesetzt wird. Unter anderem weist UHMWPE eine hohe mechanische Festigkeit und Härte, einen niedrigen Reibungskoeffizienten und eine hohe Verschleißfestigkeit auf. Um insbesondere die Verschleißfestigkeit und damit die Lebensdauer der Implantate zu erhöhen, ist es üblich, das UHMWPE durch die Einwirkung von Betastrahlung und/oder Gammastrahlung zu vernetzen.

Da die Strahlungsvernetzung über einen radikalischen Reaktionsmechanismus erfolgt, verbleiben nach der Bestrahlung in dem UHMWPE freie Radikale, die das Material anfällig für Oxidationsprozesse machen und somit die Produkteigenschaften wiederum negativ beeinflussen können. Um dieses Problem zu vermeiden, ist es aus dem Stand der Technik ebenfalls bekannt, das UHMWPE mit einem Antioxidans, wie z. B. α-Tocopherol (Vitamin E) zu versetzen, um die nach der Vernetzung verbleibenden freien Radikale abzufangen.

Derartige Verfahren zur Herstellung von vernetzten Formkörpern aus UHMWPE unter Verwendung von Beta- oder Gammastrahlung sind z. B. in der internationalen Patentanmeldung WO 2007/019874 A1 beschrieben.

Beide Arten der verwendeten ionisierenden Strahlung sind jedoch mit bestimmten Nachteilen verbunden. Im Fall der Betastrahlung, bei der es sich um eine Teilchenstrahlung handelt, ist dies in erster Linie die begrenzte Eindringtiefe in den UHMWPE-Formkörper von maximal ca. 40 mm, so dass eine durchgängig homogene Vernetzung von größeren Formkörpern nicht möglich ist. Die bestrahlten Formkörper (z. B. Rohlinge in Form von Stangen oder Riegeln) weisen jedoch meist aus technischen Gründen in einer Raumrichtung eine größere Abmessung auf, so dass der nicht vernetzte Abschnitt jeweils verworfen werden muss, was entsprechend unwirtschaftlich ist.

Bei der Gammastrahlung entfällt zwar das Problem der begrenzten Eindringtiefe, äußerst nachteilig ist hier jedoch die im Vergleich zur Betastrahlung wesentlich geringere Dosisleistung der verfügbaren Strahlungsquellen (radioaktive Isotope wie z. B. Cobalt-60). Dadurch sind längere Bestrahlungszeiten erforderlich, um die gewünschte Dosis zu erreichen, was ebenfalls wirtschaftlich nachteilig ist, u.a. auch weil keine Warmbestrahlung möglich ist. Ein weiterer Nachteil von Gammastrahlung ist die eingeschränkte Reproduzierbarkeit bzw. Steuerbarkeit der Strahlungsleistung.

Der Erfindung liegt daher die Aufgabe zugrunde, ein Verfahren zur Herstellung eines vernetzten Formkörpers aus UHMWPE vorzuschlagen, bei dem die genannten Probleme ganz oder teilweise vermieden werden.

Diese Aufgabe wird bei dem Verfahren der eingangs genannten Art erfindungsgemäß dadurch gelöst, dass die Bestrahlung des Formkörpers mit Röntgenstrahlung erfolgt.

Bei Verwendung von Röntgenstrahlung zur Vernetzung des UHMWPE-Formkörpers können ausreichend hohe Dosisleistungen erreicht werden, so dass kürzere Bestrahlungszeiten und eine Warmbestrahlung möglich sind. Zudem sind die Steuerung und Reproduzierbarkeit der Strahlungsleistung bei Röntgenstrahlung unproblematisch. Für eine gleichbleibende Produktqualität ist dies gerade bei medizinischen Produkten von großer Bedeutung. Andererseits weist Röntgenstrahlung im Vergleich zu Betastrahlung eine wesentlich höhere Eindringtiefe auf, die zumindest bei den in der Praxis relevanten Abmessungen der zu bestrahlenden Formkörper nicht limitierend ist.

Das erfindungsgemäße Verfahren vereint somit wesentliche Vorteile, die bei Verwendung von Betastrahlung oder Gammastrahlung jeweils nur einzeln erzielt werden können.

Als Antioxidans, mit dem das UHMWPE versetzt ist, können verschiedene Antioxidationsmittel eingesetzt werden, die für eine Verwendung auch im medizinischen Bereich zugelassen sind. Bevorzugt ist das Antioxidans ausgewählt aus Tocopherolen, Tocotrienolen, Ascorbinsäure, polyphenolischen Antioxidantien wie z. B. Flavonoiden, Butylhydroxytoluol (BTH) und Butylhydroxyanisol (BTA).

Bei einer bevorzugten Ausführungsform der Erfindung ist das Antioxidans α-Tocopherol. Dieses wird auch als Vitamin E bezeichnet, wobei der Begriff Vitamin E in einem weiteren Sinne alle Tocopherole, Tocotrienole und weitere fettlösliche Antioxidantien umfasst.

Der Formkörper, der als Ausgangspunkt für das erfindungsgemäße Verfahren dient, ist bevorzugt hergestellt durch Mischen von pulverförmigem und granularem UHMWPE mit dem Antioxidans und anschließendem Verdichten der Mischung, insbesondere mittels Formpressen oder Ram-Extrusion. Aufgrund der hohen Schmelzviskosität von UHMWPE ist eine Verarbeitung mittels herkömmlichem Verfahren zur Bearbeitung von Kunststoffen (z. B. Spritzgießen) in der Regel nicht möglich oder nicht praktikabel.

Der Anteil des Antioxidans in dem Formkörper liegt bevorzugt im Bereich von 0,02 bis 2 Gew.%, weiter bevorzugt von 0,05 bis 0,5 Gew.%, und insbesondere bei ca. 0,1 Gew.%. Diese Menge hat sich als ausreichend erwiesen, um die nachteiligen Wirkungen von freien Radikalen weitgehend zu verhindern.

UHMWPE wird durch das Ziegler-Natta-Verfahren unter Verwendung geeigneter Katalysatoren hergestellt. Das im Rahmen der Erfindung eingesetzte UHMWPE weist typischerweise ein Molekulargewicht im Bereich von 5.10⁶ bis 10⁷ g/mol auf (ermittelt aus der intrinsischen Viskosität) und eine Dichte im Bereich von 0,92 bis 0,94 g/cm³. Geeignete Typen von UHMWPE in Pulverform sind z. B. von der Ticona GmbH unter den Bezeichnungen GUR 1020 und GUR 1050 erhältlich, bzw. als Mischung mit 0,1 Gew.% α-Tocopherol versetzt unter den Bezeichnungen GUR 1020-E und GUR 1050-E.

In der Regel weist der Formkörper aus dem mit Antioxidans versetzen UHMWPE in jeder Raumrichtung eine Abmessung von 50 mm oder mehr auf, und bevorzugt in einer Raumrichtung von 100 mm oder mehr. Formkörper mit entsprechenden Abmessungen können als Rohlinge für die Herstellung von Implantaten oder Implantatteilen verwendet werden, wobei die Eindringtiefe der Röntgenstrahlung, wie bereits oben beschrieben, in diesem Bereich nicht limitierend ist, so dass eine durchgehend homogene Vernetzung des UHMWPE gewährleistet ist.

Die Obergrenzen für die Abmessungen des Formkörpers ergeben sich somit nur aus den konstruktiven Gegebenheiten der Bestrahlungsvorrichtung, wobei es für eine möglichst wirtschaftliche Prozessführung günstig ist, den in der Vorrichtung zu Verfügung stehenden Raum mit einer Mehrzahl von Formkörpern optimal auszunutzen.

Der Formkörper wird bei dem erfindungsgemäßen Verfahren auf eine Temperatur von 90 °C oder mehr aufgeheizt, so dass die nachfolgende Bestrahlung bei einer erhöhten Temperatur des Formkörpers erfolgt. Durch diese Erwärmung wird die Beweglichkeit der Molekülketten des UHMWPE erhöht und damit auch die Reaktionsgeschwindigkeit der Vernetzung über die mittels der Strahlung erzeugten Radikale. Die Effektivität der Strahlungsvernetzung wird dadurch erhöht. Dabei ist es im Rahmen der Erfindung aufgrund der vorgesehenen Bestrahlungszeiten (siehe unten) ausreichend, den Formkörper vorzuwärmen, so dass er trotz einer gewissen Abkühlung während der gesamten Zeitdauer der Bestrahlung eine ausreichend hohe Temperatur behält. Auf eine Beheizung während der Bestrahlung, die einen zusätzlichen apparativen Aufwand bedeutet, kann hingegen verzichtet werden. Dies stellt einen weiteren Vorteil von kürzeren Bestrahlungszeiten (insbesondere im Vergleich zur Gammabestrahlung) dar.

Vorzugsweise erfolgt das Aufheizen des Formkörpers derart, dass der Formkörper während des Bestrahlens eine Temperatur von 90 °C oder mehr behält. Welche Aufheiztemperatur hierfür erforderlich ist, hängt von verschiedenen Einflussfaktoren, insbesondere auch von den Abmessungen des Formkörpers, ab. Damit der Formkörper möglichst langsam abkühlt, ist es bevorzugt, wenn er während des Bestrahlens in einer thermischen Isolierung angeordnet ist.

Bei einer bevorzugten Ausführungsform der Erfindung erfolgt das Aufheizen auf eine Temperatur unterhalb des Schmelzpunktes des UHMWPE, die bei ca. 135 °C liegt. Bevorzugt wird der Formkörper auf eine Temperatur von 105 bis 130 °C aufgeheizt, weiter bevorzugt von 110 bis 120 °C. In letzterem Fall sinkt die Temperatur des Formkörpers während der Bestrahlung typischerweise auf etwa 100 °C, was für eine effektive Vernetzung in der Regel ausreichend ist.

Bei einer weiteren Ausführungsform der Erfindung wird der Formkörper auf eine Temperatur im Bereich oder oberhalb des Schmelzpunktes des UHMWPE aufgeheizt, bevorzugt auf eine Temperatur von 135 bis 160 °C. Die Molekülbeweglichkeit ist oberhalb des Schmelzpunktes naturgemäß nochmals deutlich erhöht. Allerdings kann diese Vorgehensweise unter Umständen auch zu einer Beeinträchtigung der mechanischen Eigenschaften des Formkörpers führen. Gemäß einer vorteilhaften Ausführungsform der Erfindung wird der Formkörper mit einer Strahlungsdosis von 60 bis 110 kGy bestrahlt, bevorzugt von 65 bis 100 kGy, weiter bevorzugt von 70 bis 90 kGy. Entsprechende Strahlungsdosen haben sich auch bei Verwendung von Betastrahlung als ausreichend erwiesen.

Günstigerweise wird die Bestrahlung mit einer Dosisrate von 10 bis 30 kGy/h durchgeführt, bevorzugt von 15 bis 25 kGy/h. Eine ausreichende Röntgenleistung, um diese Dosisrate zu erreichen, kann z. B. mittels eines Rhodotrons erzeugt werden.

Unter Berücksichtigung der oben genannten Strahlungsdosen und Dosisraten ergibt sich für das erfindungsgemäße Verfahren ein Bestrahlungszeitraum, der typischerweise im Bereich von 3 bis 7 Stunden liegt, bevorzugt im Bereich von 4 bis 5 Stunden. Demgegenüber sind bei der Verwendung von Gammastrahlung in der Regel Bestrahlungszeiten von 16 bis 24 Stunden erforderlich.

Der vernetzte Formkörper aus UHMWPE dient vorzugsweise als Rohling für die Herstellung eines Implantats oder Implantatteils durch zerspanende Bearbeitung, insbesondere durch Drehen und Fräsen.

Gegenstand der vorliegenden Erfindung ist daher auch ein Verfahren zur Herstellung eines Implantats oder eines Implantatteils, insbesondere eines Inlays für ein künstliches Hüftgelenk, umfassend die Durchführung des erfindungsgemäßen Verfahrens zur Herstellung eines vernetzten Formkörpers aus UHMWPE und die zerspanende Bearbeitung des vernetzten Formkörpers.

Günstigerweise wird der Formkörper zwischen der Bestrahlung und der zerspanenden Bearbeitung keiner thermischen Nachbehandlung unterworfen. Wenn die Bestrahlung bei einer ausreichend hohen Temperatur durchgeführt wird, ist eine solche thermische Nachbehandlung (Annealing) zur Erzielung eines hohen Vernetzungsgrades nicht erforderlich und kann im Gegenteil die mechanischen Eigenschaften des Formkörpers beeinträchtigen.

Das hergestellte Implantat oder Implantatteil kann anschließend verpackt und mittels Ethylenoxid sterilisiert werden.

### Beispiel:

### 1. Herstellung von Formkörpern

Ausgangspunkt für die Herstellung der Formkörper ist pulverförmiges UHMWPE (GUR 1020-E, Ticona GmbH), das gemäß der ISO 5834-1:2005 (UHMWPE in Pulverform für chirurgische Implantate) hergestellt ist und das mit 0,1 Gew.% α-Tocopherol (Vitamin E) versetzt ist. Aus dieser Mischung werden plattenförmige Halbzeuge mittels Formpressen hergestellt (Presssinterverfahren bei einer Temperatur von ca. 220 °C und einem Druck von ca. 50 bar). Anschließend werden die Platten bei ca. 110 °C für 15 bis 25 Stunden getempert.

Aus den Platten werden Formkörper für die Durchführung des erfindungsgemäßen Verfahrens in Form von Riegeln mit folgenden Abmessungen ausgesägt: 50·50·940 mm, 55·55·940 mm und 60·60·940 mm.

### 2. Aufheizen der Formkörper

Die Formkörper werden über einen Zeitraum von 10 bis 20 Stunden bei einer Temperatur von 115 °C aufgeheizt, mit dem Ziel, dass die Temperatur während der nachfolgenden Bestrahlung im Bereich von ca. 100 °C liegt. Die relativ lange Aufheizdauer wird gewählt, um sicherzustellen, dass die Aufheiztemperatur auch im Inneren der Formkörper vollständig erreicht wird. Da die Aufheiztemperatur deutlich unterhalb der Schmelztemperatur des UHMWPE liegt, ist eine längere Temperierung nicht mit negativen Auswirkungen verbunden.

### 3. Bestrahlen der Formkörper

Die Formkörper werden in einer thermischen Isolierung in eine Röntgenbestrahlungsvorrichtung eingebracht und während eines Zeitraums von ca. vier Stunden mit einer Dosisrate von ca. 20 kGy/h bestrahlt. Die von den Formkörpern aufgenommene Strahlendosis liegt somit bei ca. 80 kGy.

Die Temperatur der Formkörper liegt während des gesamten Bestrahlungszeitraums bei ca. 100 °C oder mehr. Nach der Bestrahlung werden die Formkörper in der thermischen Isolierung langsam abkühlen gelassen.

### 4. Herstellung von Implantatteilen

Aus den verschiedenen Formteilen (Riegeln) werden zunächst durch Drehen Stangen mit einer Länge von 940 mm und einem Durchmesser von 50, 55 bzw. 60 mm hergestellt. Aus diesen Stangen werden durch weitere zerspanende Bearbeitung beispielsweise Inlays für künstliche Hüftgelenke hergestellt. Die Hüftinlays werden verpackt und mittels Ethylenoxid sterilisiert.

## Patentansprüche

1. Verfahren zur Herstellung eines vernetzten Formkörpers aus UHMWPE, umfassend die Schritte:
- Bereitstellen eines Formkörpers aus UHMWPE, welches mit einem Antioxidans versetzt ist;
- Aufheizen des Formkörpers auf eine Temperatur von 100 °C oder mehr; und
- Bestrahlen des Formkörpers, um das UHMWPE in dem Formkörper zu vernetzen,
**dadurch gekennzeichnet, dass** die Bestrahlung des Formkörpers mit Röntgenstrahlung erfolgt.

2. Verfahren nach Anspruch 1, wobei das Antioxidans ausgewählt ist aus Tocopherolen, Tocotrienolen, Ascorbinsäure, polyphenolischen Antioxidantien wie z.B. Flavonoiden, Butylhydroxytoluol und Butylhydroxyanisol, und wobei das Antioxidans bevorzugt α-Tocopherol ist.

3. Verfahren nach einem der vorhergehenden Ansprüche, wobei der Formkörper hergestellt ist durch Mischen von pulverförmigem oder granularem UHMWPE mit dem Antioxidans und anschließendem Verdichten der Mischung, insbesondere mittels Formpressen oder Ram-Extrusion.

4. Verfahren nach einem der vorhergehenden Ansprüche, wobei der Anteil des Antioxidans in dem Formkörper im Bereich von 0,02 bis 2 Gew.% liegt, bevorzugt von 0,05 bis 0,5 Gew.%, insbesondere bei ca. 0,1 Gew.%.

5. Verfahren nach einem der vorhergehenden Ansprüche, wobei das UHMWPE ein Molekulargewicht im Bereich von 5*10⁶ bis 10⁷ g/mol und eine Dichte im Bereich von 0,92 bis 0,94 g/cm³ aufweist.

6. Verfahren nach einem der vorhergehenden Ansprüche, wobei der Formkörper in jeder Raumrichtung eine Abmessung von 50 mm oder mehr aufweist, und bevorzugt in einer Raumrichtung von 100 mm oder mehr.

7. Verfahren nach einem der vorhergehenden Ansprüche, wobei das Aufheizen des Formkörpers derart erfolgt, dass der Formkörper während des Bestrahlens eine Temperatur von 90 °C oder mehr behält.

8. Verfahren nach einem der vorhergehenden Ansprüche, wobei der Formkörper während des Bestrahlens in einer thermischen Isolierung angeordnet ist.

9. Verfahren nach einem der vorhergehenden Ansprüche, wobei das Aufheizen auf eine Temperatur unterhalb des Schmelzpunktes des UHMWPE erfolgt, bevorzugt auf eine Temperatur von 105 bis 130 °C, insbesondere von 110 bis 120 °C.

10. Verfahren nach einem der Ansprüche 1 bis 8, wobei das Aufheizen auf eine Temperatur im Bereich oder oberhalb des Schmelzpunktes des UHMWPE erfolgt, bevorzugt auf eine Temperatur von 135 bis 160 °C.

11. Verfahren nach einem der vorhergehenden Ansprüche, wobei der Formkörper mit einer Strahlungsdosis von 60 bis 110 kGy, bevorzugt von 65 bis 100 kGy, weiter bevorzugt von 70 bis 90 kGy, bestrahlt wird.

12. Verfahren nach einem der vorhergehenden Ansprüche, wobei die Bestrahlung mit einer Dosisrate von 10 bis 30 kGy/h durchgeführt wird, bevorzugt von 15 bis 25 kGy/h.

13. Verfahren nach einem der vorhergehenden Ansprüche, wobei die Bestrahlung während eines Zeitraums von 3 bis 7 Stunden, bevorzugt von 4 bis 5 Stunden, durchgeführt wird.

14. Verfahren zur Herstellung eines Implantats oder eines Implantatteils, insbesondere eines Inlays für ein künstliches Hüftgelenk, umfassend die Durchführung des Verahrens nach einem der vorhergehenden Ansprüche und die zerspanende Bearbeitung des vernetzten Formkörpers.

15. Verfahren nach Anspruch 14, wobei der Formkörper zwischen der Bestrahlung und der zerspanenden Bearbeitung keiner thermischen Nachbehandlung unterworfen wird; und/oder wobei das Implantat oder Implantatteil verpackt und mittels Ethylenoxid sterilisiert wird.

## Claims

1. Method for producing a cross-linked moulded body made of UHMWPE, comprising the steps:
- providing a moulded body made of UHMWPE to which an antioxidant has been added;
- heating the moulded body to a temperature of 100 °C or more; and
- irradiating the moulded body to cross-link the UHMWPE in the moulded body,
**characterized in that** the irradiation of the moulded body is done with X-rays.

2. Method according to claim 1, wherein the antioxidant is selected from tocopherols, tocotrienols, ascorbic acid, phenolic antioxidants such as, e.g., flavenoids, butylated hydroxytoluene and butylated hydroxyanisole, and wherein the antioxidant is preferably α-tocopherol.

3. Method according to any one of the preceding claims, wherein the moulded body is produced by mixing of powdered or granular UHMWPE with the antioxidant and subsequent compacting of the mixture, in particular by compression moulding or ram extrusion.

4. Method according to any one of the preceding claims, wherein the proportion of the antioxidant in the moulded body is in the range from 0.02 to 2 wt.%, preferably from 0.05 to 0.5 wt.%, in particular at about 0.1 wt.%.

5. Method according to any one of the preceding claims, wherein the UHMWPE has a molecular weight in the range from 5*10⁶ to 10⁷ g/mol and a density in the range from 0.92 to 0.94 g/cm³.

6. Method according to any one of the preceding claims, wherein the moulded body has a size in each dimension of 50 mm or more, and preferably in each dimension of 100 mm or more.

7. Method according to any one of the preceding claims, wherein the heating of the moulded body is performed in such a way that the moulded body maintains a temperature of 90 °C or more during the irradiation.

8. Method according to any one of the preceding claims, wherein the moulded body is placed within a thermal insulation during the irradiation.

9. Method according to any one of the preceding claims, wherein the heating is performed up to a temperature below the melting point of the UHMWPE, preferably up to a temperature of 105 to 130 °C, in particular from 110 to 120 °C.

10. Method according to any one of claims 1 to 8, wherein the heating is performed up to a temperature in the range of or above the melting point of the UHMWPE, preferably up to a temperature from 135 to 160 °C.

11. Method according to any one of the preceding claims, wherein the moulded body is irradiated with a radation dose from 60 to 110 kGy, preferably from 65 to 100 kGy, more preferably from 70 to 90 kGy.

12. Method according to any one of the preceding claims, wherein the irradiation is performed with a dose rate from 10 to 30 kGy/h, preferably from 15 to 25 kGy/h.

13. Method according to any one of the preceding claims, wherein the irradiation is performed during a time period from 3 to 7 hours, preferably from 4 to 5 hours.

14. Method for producing an implant or an implant part, in particular of an inlay for an artificial hip joint, comprising the execution of the method according to any one of the preceding claims, and the processing of the cross-linked moulded body by chip removal.

15. Method according to claim 14, wherein the moulded body is not subjected to a thermal post-treatment between the irradiation and the processing by chip removal; and/or wherein the implant or implant part is packaged and sterilized by ethylene oxide.

## Revendications

1. Procédé de fabrication d'un corps moulé réticulé à partir de UHMWPE, comprenant les étapes :
- de fourniture d'un corps moulé de UHMWPE, lequel est mélangé à un antioxydant ;
- de chauffage du corps moulé à une température de 100 °C ou plus ; et
- d'exposition du corps moulé à un rayonnement pour réticuler le UHMWPE dans le corps moulé,
**caractérisé en ce que** l'exposition du corps moulé à des rayons X est effectuée.

2. Procédé selon la revendication 1, dans lequel l'antioxydant est choisi parmi des tocophérols, des tocotriénols, de l'acide ascorbique, des antioxydants polyphénoliques comme par exemple des flavonoïdes, le butylhydroxytoluol et le butylhydroxyanisol, et dans lequel l'antioxydant est de manière préférée de l'α-tocophérol.

3. Procédé selon l'une des revendications précédentes, dans lequel le corps moulé est fabriqué en mélangeant de l'UHMWPE pulvérulent ou granulaire à l'antioxydant puis en compactant le mélange, notamment au moyen d'un pressage de moulage ou d'une extrusion Ram.

4. Procédé selon l'une des revendications précédentes, dans lequel la fraction de l'antioxydant dans le corps moulé se situe dans la plage de 0,02 à 2 % en poids, de manière préférée de 0,05 à 0,5 % en poids, notamment est de l'ordre d'environ 0,1 % en poids.

5. Procédé selon l'une des revendications précédentes, dans lequel le UHMWPE présente un poids moléculaire dans la plage de 5*10⁶ à 10⁷ g/mole et une densité dans la plage de 0,92 à 0,94 g/cm³.

6. Procédé selon l'une des revendications précédentes, dans lequel le corps moulé présente dans chaque direction spatiale une dimension de 50 mm ou plus, et de manière préférée dans une direction spatiale de 100 mm ou plus.

7. Procédé selon l'une des revendications précédentes, dans lequel le chauffage du corps moulé est effectué de telle manière que le corps moulé conserve au cours de l'exposition au rayonnement une température de 90 °C ou plus.

8. Procédé selon l'une des revendications précédentes, dans lequel le corps moulé est disposé au cours de l'exposition au rayonnement dans une isolation thermique.

9. Procédé selon l'une des revendications précédentes, dans lequel le chauffage est effectué à une température sous le point de fusion du UHMWPE, de manière préférée à une température de 105 à 130 °C, notamment de 110 à 120 °C.

10. Procédé selon l'une des revendications 1 à 8, dans lequel le chauffage est effectué à une température dans la plage ou au-dessus du point de fusion du UHMWPE, de manière préférée à une température de 135 à 160 °C.

11. Procédé selon l'une des revendications précédentes, dans lequel le corps moulé est exposé au rayonnement à une dose de rayonnement de 60 à 110 kGy, de manière préférée de 65 à 100 kGy, de manière davantage préférée de 70 à 90 kGy.

12. Procédé selon l'une des revendications précédentes, dans lequel l'exposition au rayonnement est mise en œuvre à un débit de dose de 10 à 30 kGy/h, de manière préférée de 15 à 25 kGy/h.

13. Procédé selon l'une des revendications précédentes, dans lequel l'exposition au rayonnement est mise en œuvre au cours d'un intervalle de temps de 3 à 7 heures, de manière préférée de 4 à 5 heures.

14. Procédé de fabrication d'un implant ou d'une partie d'implant, en particulier d'un inlay pour une articulation artificielle de la hanche, comprenant la mise en œuvre du procédé selon l'une des revendications précédentes et l'usinage par enlèvement de copeaux du corps moulé réticulé.

15. Procédé selon la revendication 14, dans lequel le corps moulé n'est soumis à l'action d'aucun traitement ultérieur thermique entre l'exposition au rayonnement et l'usinage par enlèvement de copeaux ; et/ou dans lequel l'implant ou la partie d'implant est emballé et stérilisé au moyen d'un oxyde d'éthylène.
